## Europäisches Patentamt

## European Patent Office

## Office européen des brevets

(11) Veröffentlichungsnummer: **0 137 433**
**B1**

(12) # EUROPÄISCHE PATENTSCHRIFT

(45) Veröffentlichungstag der Patentschrift:
12.08.87

(51) Int. Cl.⁴: **C 07 C 29/10,** C 07 C 31/02, C 07 C 31/10, C 07 C 31/12

(21) Anmeldenummer: **84111683.3**

(22) Anmeldetag: **29.09.84**

(54) **Verfahren zur kontinuierlichen Herstellung von Alkoholen.**

(30) Priorität: **06.10.83 DE 3336644**

(43) Veröffentlichungstag der Anmeldung:
**17.04.85 Patentblatt 85/16**

(45) Bekanntmachung des Hinweises auf die Patenterteilung:
**12.08.87 Patentblatt 87/33**

(84) Benannte Vertragsstaaten:
**AT BE DE FR GB IT NL SE**

(56) Entgegenhaltungen:
DE-A-2 128 683
FR-A-1 038 814

**Die Akte enthält technische Angaben, die nach dem Eingang der Anmeldung eingereicht wurden und die nicht in dieser Patentschrift enthalten sind.**

(73) Patentinhaber: **DEUTSCHE TEXACO AKTIENGESELLSCHAFT, Überseering 40, D-2000 Hamburg 60 (DE)**

(72) Erfinder: **Neier, Wilhelm, An der Landwehr 40, D-4134 Rheinberg 3 (DE)**
Erfinder: **Webers, Werner, Rektor- Horn- Strasse 42, D-4134 Rheinberg 3 (DE)**
Erfinder: **Dettmer, Michael, Königsbergerstrasse 29, D-4134 Rheinberg 3 (DE)**

(74) Vertreter: **Schupfner, Gerhard D., Müller, Schupfner & Gauger Karlstrasse 5 Postfach 14 27, D-2110 Buchholz/Nordheide (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

## Beschreibung

Die vorliegende Erfindung betrifft ein Verfahren zur kontinuierlichen Herstellung von Alkoholen mit 1 bis 5 Kohlenstoffatomen durch Spaltung von Äthern der allgemeinen Formel

R - O - R$_1$,

worin R und R$_1$ jeweils einen Alkylrest mit 1 bis 5 Kohlenstoffatomen bedeuten, und solchen Äthergemischen in Gegenwart eines sauren Katalysators bei erhöhter Temperatur und erhöhtem Druck.

Ätherspaltreaktionen werden üblicherweise zur Gewinnung reiner Olefine durchgeführt. Hierzu gibt es zahlreiche Veröffentlichungen, bei welchen in der Regel zunächst aus olefinhaltigen Gemischen mit Alkoholen in Gegenwart von Ionenaustauschern Äther hergestellt und diese dann in Gegenwart eines sauren Katalysators bei erhöhter Temperatur unter Bildung des reinen Olefins und des Alkohols gespalten werden. Gemäß DE-OS 28 02 199, EP 3305, wird beispielsweise auf diese Weise aus C$_4$-Kohlenwasserstoffströmen mit Hilfe von C$_3$, C$_4$-Alkoholen über den jeweiligen Tert.butyläther reines Isobuten gewonnen.

Bei den Alkoholsynthesen durch saure Hydratation von Olefinen entsteht als unerwünschtes Nebenprodukt der entsprechende Äther. Hierbei ist bekannt, daß durch Rückführung der von den Alkoholen destillativ abgetrennten Äther deren Bildung zurückgedrängt werden kann. So wurde bereits in US-PS 2 050 445 für die Äthanolherstellung in einem kontinuierlichen Verfahren mit wäßriger Phosphorsäure als Katalysator davon berichtet (Seite 3, linke Spalte, Zeile 44 - 54), daß durch Zurückführung von Diäthyläther mit dem Umlaufgas die weitere Bildung von Äther zurückgedtängt werden kann. Die Rückführung von Diisopropyläther zum Umlaufgas der Isopropylalkohol-Synthese aus Propylen ist in der CA-PS 867 797 beschrieben. Es wird also bereits seit längerer Zeit zur Erhöhung der Ausbeute in den Äthanol- und Ispropylalkohol-Synthesen der entsprechende Äther in der erzeugten Menge zum Umlaufgas zurückgegeben.

Auch in der DE-OS 27 59 237 wird beschrieben, daß die gebildeten Nebenprodukte dem Einsatzprodukt vor Eintritt in den Reaktor wieder zugeführt werden.

Eine gänzliche Unterdrückung der Ätherbildung durch eine Ätherrückführung in den Einsatzstrom zum Reaktor ist mit erheblichen Nachteilen verbunden. So nimmt die Raumzeitausbeute nach eigenen Versuchen, z. B. bei der IPA-Synthese, abhängig von der Konzentration des eingesetzten Propylens, durch diese Maßnahme um 30 % bis 50 % ab. Des weiteren müssen bei großen Gaskreislaufmengen, das heißt bei geringem Gasumsatz per pass, erhebliche Mengen Äther im Kreislauf geführt werden. Die zusätzliche Verdampfung des Äthers im Einsatzstrom führt zu einem erhöhten Energieverbrauch.

Als alternative Möglichkeit ist eine separate Ätherspaltung durch Überleiten des Äthers im Dampfzustand über Aluminiumoxid anzusehen, siehe Houben-Weyl, Methoden der Organischen Chemie VI/3 (1965), 143. Die Umsetzung von Diisopropyläther ergibt aber hauptsächlich Propen und nur geringe Mengen Alkohol.

Aus DE-A-2 128 683 ist ein Verfahren bekannt, wonach bei der Herstellung von Methanol durch katalytische Umsetzung von Kohlenmonoxiden mit Wasserstoff entstehendes Dimethyläther nach dessen Abtrennung in einer Destillationszone mit Wasserdampf in Gegenwart von γ-Aluminiumoxid bei Temperaturen von 200° bis 300° C behandelt und das Methanol enthaltende Reaktionsgemisch in die Destillationszone zurückgeführt wird.

Gemäß FR-A-1 038 814 wird Di-sec-butyläther in flüssiger Phase mit wäßriger 70 bis 78 %iger Schwefelsäure bei 50° bis 75° C hydratisiert.

Der Erfindung liegt die Aufgabe zugrunde, ein solches Verfahren bereitzustellen, das es gestattet, einen Äther direkt und quantitativ in den bzw. die entsprechenden Alkohole umzuwandeln.

Die Aufgabe wird erfindungsgemäß durch ein Verfahren gelöst, das dadurch gekennzeichnet ist, daß Äther der allgemeinen Formel

R - O - R$_1$,

worin R und R$_1$ Alkylgruppen mit 1 bis 5 Kohlenstoffatome bedeuten, mit überschüssigem Wasser bei einer Temperatur von 100 bis 180° C und einem Druck von 10 bis 100 bar in Gegenwart eines üblichen sauren Hydratationskatalysators in Kontakt gebracht, die fluide Ätherphase im Kreislauf geführt und die wäßrige, den gebildeten Alkohol enthaltende Phase aus dem Reaktor abgezogen und zur Gewinnung des Alkohols in an sich bekannter Weise aufgearbeitet wird.

Nach einer bevorzugten Ausführungsform des erfindungsgemäßen Verfahrens wird aus der fluiden Ätherphase vor deren Rückführung in den Spaltreaktor der darin enthaltene Alkohol durch Extraktion insbesondere mit Wasser entfernt.

Das erfindungsgemäße Verfahren gestattet nun die direkte und quantitative Umwandlung eines Äthers in Alkohole. Aus dem Verfahren wird im wesentlichen nur die den gebildeten Alkohol aufnehmende wäßrige Phase abgezogen. Aus dieser alkoholischen Lösung kann dann in einem nachgeschaltetem Verfahrensschritt der Alkohol (neben einer geringen Menge an gelöstem Äther) in bekannter Weise destillativ abgetrennt werden.

Bei der Durchführung des erfindungsgemäßen Verfahrens erhält man überraschenderweise praktisch kein Olefin. Aus dem Reaktionssystem wird kein Gas entnommen. Bei der Spaltung von Diisopropyläther befinden sich lediglich 2 bis 3 % Propylen im Gleichgewicht in der organischen Phase gelöst, die ohne Abtrennung mit dem Ätherstrom ständig in den Reaktor zurückgeführt werden.

Die beigefügten Zeichnungen erläutern Ausführungsbeispiele des erfindungsgemäßen Verfahrens. Es zeigen

Fig. 1 eine mögliche Schaltung des Reaktors als Sumpfphasenreaktor,

Fig. 2 ein Verfahrensschema unter Einbeziehung eines Extraktors.

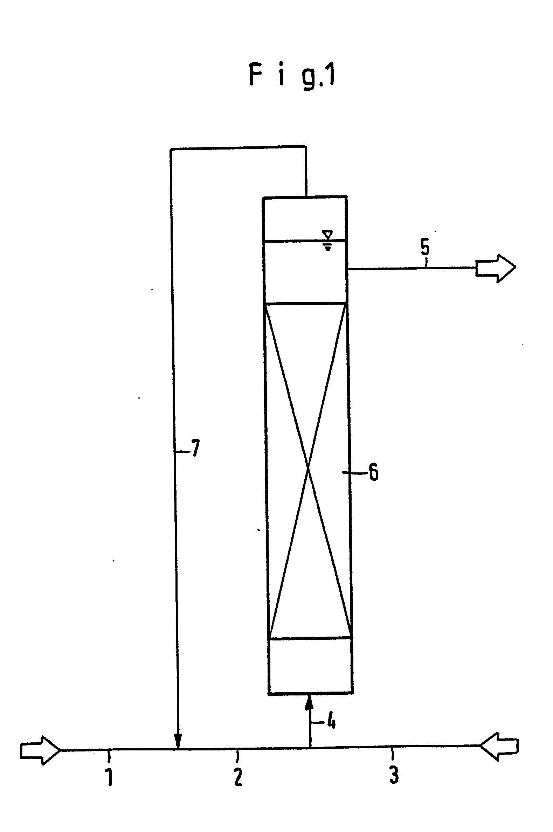

Gemäß Figur 1 werden Äther über Leitung 1 und Wasser über Leitung 3 gemeinsam über Leitung 4 kontinuierlich in einen mit einem Ionenaustauscher gefüllten Rohrreaktor 6 dosiert. In einer säurekatalytischen Mehrphasenreaktion bei erhöhtem Druck und erhöhter Temperatur wird dabei der Äther hydrolisiert. Der gebildete Alkohol sowie die beiden Edukte verteilen sich in zwei nicht mischbaren Flüssigphasen, Alkohol/Wasser und Äther/Alkohol. Hierbei ist für das Verfahren von Nutzen, daß sich der Alkohol wesentlich besser als der Äther in der Wasserphase löst. Nach der Phasentrennung kann somit das gewünschte Reaktionsprodukt neben einem kleinen Anteil an Äther mit Überschuß an Wasser über Leitung 5 abgezogen werden. Nicht umgesetzter Äther und ggf. geringe Olefinanteile, die sich am Reaktorkopf als obere Phase abtrennen, werden ohne Zwischenbehandlung über Leitung 7 dem Reaktor 6 erneut zugeführt. Um einen hohen Ätheranteil ( > 90 %) zu spalten, und um eine gute Durchmischung der beiden Phasen zu erreichen, wird die sich am Reaktorkopf absetzende leichterephase (Äther/Alkohol) mit relativ hoher Umlaufgeschwindigkeit in den Reaktorsumpf zurückgeführt. Es kommt somit (abgesehen von dem mit dem Produktwaser abgezogenen Äther, der nach Destillation auch wieder zurückgeführt werden kann) zu einem vollständigen Umsatz des eingespeisten Äthers zu Alkohol, da auch die rückgeführten Olefine unter Reaktionsbedingungen zu Alkoholen hydratisiert werden. Der Umsatz beträgt ohne Rückführung des mit dem Alkohol ausgetragenen Äthers 90 bis 93 % (siehe Beispiel 1). Mit Rückführung erreicht man nahezu vollständigen Umsatz.

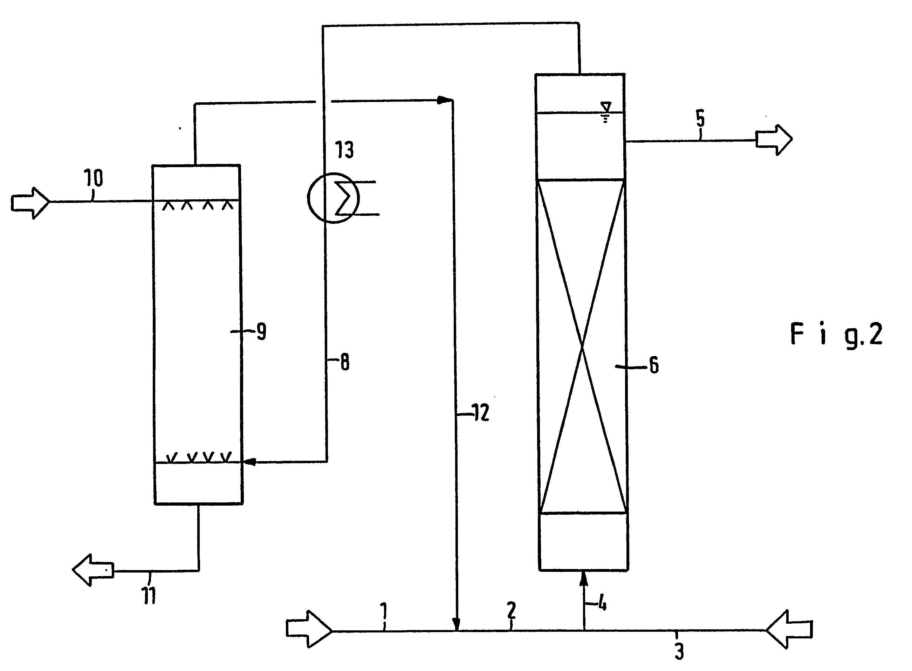

Gemäß Figur 2 wird der Rückführstrom über Leitung 8 in den Extraktor 9 geführt, worin bei tieferer Temperatur, insbesondere bei 80 bis 100° C, die noch im Rückführstrom enthaltenen relativ hohen Anteile Alkohol mit Wasser ausgewaschen werden. Der vom Alkohol befreite Rückführstrom wird sodann über Leitung 12 erneut erwärmt und in den Spaltreaktor zurückgeführt.

Als Katalysator für die Ätherspaltung wird ein für das Verfahren der Hydratation von Olefinen üblicher flüssiger und insbesondere fester saurer Katalysator eingesetzt. Besonders bevorzugt hierfür sind temperaturbeständige Ionenaustauscher vom Sulfonsäuretyp.

Die Temperatur im Spaltreaktor liegt bei 100 bis 180° C, wobei im Falle des Einsatzes von organischen Ionenaustauschern die obere Temperaturgrenze durch die Temperaturbeständigkeit des Katalysators festgelegt ist. Der Druck liegt im Bereich von 10 bis 100 bar. Das Verfahren zur Ätherspaltung ist weitgehend druckunabhängig. Der Druck dient hauptsächlich zur Aufrechterhaltung der beiden Phasen im flüssigen Zustand.

Bei der erfindungsgemäßen Ätherspaltung ist ein Molverhältnis von Äther zu Wasser erforderlich, das höher als 1 Mol Äther zu 3 Mol Wasser liegt. Insbesondere liegt das Molverhältnis über 1 Mol Äther zu 10 Mol Wasser und besonders bevorzugt im Bereich von etwa 1 Mol Äther zu 50 bis 100 Mol Wasser, jeweils auf die Einsatzströme bezogen.

Die Raumzeitausbeute beträgt 1 bis 2 Mol Alkohol/ 1 Kat.h, und falls der Rückführstrom durch Extraktion mit Wasser vom Alkohol befreit wird, bis zu 3,5 Mol/l Kat.h und mehr.

Der Gehalt des Alkohols in der wäßrigen Phase des Stroms 5 liegt bei der DIPE-Spaltung bis zu 10 bis 15 Gew.% und darüber und der Äthergehalt in der wäßrigen Phase bei höchstens 1 Gew. %, in der Regel darunter.

Die folgenden Beispiel erläutern die Erfindung anhand von Fig. 1 und Fig. 2, worin sämtliche Prozent-Angaben in Gew.% angegeben sind.

**Beispiel 1**

In dem Reaktor 6 der Apparatur gemäß Fig. 1 von 26 mm lichter Weite und 5 m Länge, der mit 2,5 1 des stark sauren Ionenaustauschers Amberlite 252 als Katalysator gefüllt war, wurden über Leitung 1 stündlich 254 Diisopropyläther (DIPE) und über Leitung 3 2225 g entmineralisiertes Wasser eingespeist. Über Leitung 7 wurden stündlich 23 l einer im Reaktor sich ausgebildeten oberen organischen Phase, die aus 72 % nicht gespaltenem Äther, 20 % Isopropylalkohol, 5 % Wasser und 3 % Propylen bestand, zusammen mit dem Einsatzäther in den Sumpf des Reaktors 6 zurückgeführt. Im Reaktor wurde mit Hilfe einer Mantelbeheizung eine Reaktionstemperatur von 155° C eingestellt. Im Reaktor wurde ein Druck von 50 bar aufrechterhalten.

Aus dem Reaktor 6 wurden über Leitung 5 stündlich 2455 g einer wäßrigen Flüssigphase abgezogen, die 11,0 % Isopropylalkohol und 1 % Diisopropyläther enthielt. Pro Liter Katalysator und Stunde wurden 108 g Isopropylalkohol (1,8 Mol) gebildet. Es wurde keine Gasphase erhalten.

**Beispiel 2**

Der in Beispiel 1 beschriebene Versuch wurde mit der Maßgabe wiederholt, daß der Reaktionsdruck auf 25 bar abgesenkt wurde. Dabei wurden die gleichen Ergebnisse erhalten wie in Beispiel 1.

**Beispiel 3**

Beispiel 1 wurde in der Apparatur gemäß Fig. 2 mit der Maßgabe wiederholt, daß die obere organische Phase nach Abkühlung auf 100°C mittels Wärmetauscher 13 über Leitung 8 jetzt zusätzlich einem Extraktor 9 zugeführt wurde, um den Isopropylalkohol aus dem Kreislaufäther teilweise zu entfernen. Hierzu wurde die Ätherzuspeisung über Leitung 1 auf 450 g/h und die Wassereinspeisung auf 3292 g/h erhöht und der Extraktor 9 über Leitung 10 stündlich mit 2058 g Wasser beschickt. Über Leitung 5 wurden sodann 3440 g eines 5,9 %igen Isopropylalkohols erhalten, der noch 0,5 % Äther enthielt. Über Leitung 11 wurden stündlich 2361 g eines weiteren wäßrigen Isopropylalkohols erhalten. Der Gehalt an Isopropylalkohol lag in dieser Phase bei durchschnittlich 11,9 %, der Isopropyläthergehalt lag bei 0,9 %.

Pro Liter Katalysator wurden stündlich insbesamt 194 g Isopropylalkohol (3,2 Mol) erhalten.

**Patentansprüche**

1. Verfahren zur Herstellung von niederen aliphatischen Alkoholen mit 1 bis 5 Kohlenstoffatomen durch Spaltung eines Äthers der allgemeinen Formel

$R - O - R_1$,

worin R und $R_1$ jeweils einen Alkylrest mit 1 bis 5 Kohlenstoffatomen bedeuten, oder eines Gemischs solcher Äther in Gegenwart eines sauren Katalysators bei erhöhter Temperatur und erhöhtem Druck, <u>dadurch gekennzeichnet,</u> daß Äther mit Wasser im Überschuß bei einer Temperatur von 100 bis 180°C und einem Druck von 10 bis 100 bar, der ausreicht, die beiden Ausgangkomponenten in flüssiger Phase zu halten, in Gegenwart eines sauren Hydratationskatalysators unter Gewinnung einer fluiden Ätherphase, die Äther und Alkohol enthält, und einer wäßrigen Phäse, die Wasser und Alkohol enthält, in Kontakt gebracht, die fluide Ätherphase im Kreislauf zum Reaktor zurückgeführt und die wäßrige Phase aus dem Reaktor abgezogen und zur Gewinnung des Alkohols in an sich bekannter Weise aufgearbeitet wird.

2. Verfahren nach Anspruch 1, <u>dadurch gekennzeichnet,</u> daß aus der fluiden Ätherphase vor deren Rückführung in den Reaktor der darin enthaltene Alkohol mit Wasser extrahiert wird.

**Claims**

1. Process for the production of lower aliphatic alcohols with 1 to 5 carbon atoms by splitting an ether of the general formula

$R - O - R_1$

where R and $R_1$ each represent an alkyl residue with 1 to 5 carbon atoms, or a mixture of such ethers, in the presence of an acidic catalyst at elevated temperature and pressure, <u>characterized by</u> contacting ether in the presence of an acidic hydration catalyst with an excess of water at a temperature of 100 to 180°C and a pressure of 10 to 100 bar which is sufficient to keep the two starting components liquid thus obtaining a fluid ether phase containing ether and alcohol as well as an aqueous phase containing water and alcohol, recycling the fluid ether phase to the reactor, and withdrawing the aqueous phase from the reactor and purifying it as known per se in order to obtain the alcohol.

2. Process according to claim 1, <u>characterized by</u> extracting with water the alcohol from the fluid ether phase prior to recycling the ether phase to the reactor.

**Revendications**

1. Un procédé de préparation continue d'alcools aliphatiques légers ayant de 1 à 5 atomes de carbone réalisé par dissociation d'éthers de la formule générale

$R - O - R_1$

dans laquelle R et $R_1$ représente chacun un résidu d'alkyle ayant de 1 à 5 atomes de carbone, ou d'un mélange de tels éthers en présence d'un catalyseur acide à une température et une pression élevées, caractérisé en ce que l'éther est mis en contact avec un excès d'eau à une température comprise entre 100 et 180°C et une pression de 10 à 100 bar qui suffit à maintenir les deux composants de charge à l'état liquide en présence d'un catalyseur d'hydratation acide, une phase d'éther fluide renfermant de l'éther et de l'alcool ainsi qu'une phase aqueuse renfermant de l'eau et de l'alcool étant formées, la phase d'éther fluide est renvoyée dans le réacteur et la phase aqueuse est soutirée du réacteur et est purifiée d'une manière connue dans le but de recueillir l'alcool.

2. Procédé selon la revendication 1, caractérisé en ce que l'alcool contenu dans la phase d'éther fluide est extrait avec de l'eau avant de renvoyer la phase d'éther dans le réacteur.

# F i g.1

Fig.2